(19) Europäisches Patentamt
European Patent Office
Office européen des brevets

(11) EP 2 359 908 A2

(12) EUROPEAN PATENT APPLICATION

(43) Date of publication:
**24.08.2011 Bulletin 2011/34**

(51) Int Cl.:
***A61Q 1/02*** *(2006.01)* ***A61Q 19/02*** *(2006.01)*
***A61K 8/81*** *(2006.01)*

(21) Application number: **11166285.4**

(22) Date of filing: **29.06.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.08.2006 FR 0653423**
**25.08.2006 US 840022 P**

(62) Document number(s) of the earlier application(s) in accordance with Art. 76 EPC:
**07786946.9 / 2 054 122**

(71) Applicant: **L'Oréal**
**75008 Paris (FR)**

(72) Inventors:
- **Cassin, Guillaume**
**91140, Villebon sur Yvette (FR)**
- **Simonnet, Jean-Thierry**
**94240, CACHAN (FR)**

(74) Representative: **Tanty, François**
**Nony**
**3, rue de Penthièvre**
**75008 Paris (FR)**

Remarks:
This application was filed on 16-05-2011 as a divisional application to the application mentioned under INID code 62.

# (54) Cosmetic composition for lightening or unifying the complexion

(57) The present invention relates to a composition comprising, in a physiologically acceptable medium, concave or annular particles, especially in the form of hollow sphere portions, the said particles comprising a matrix that is coloured in the bulk.

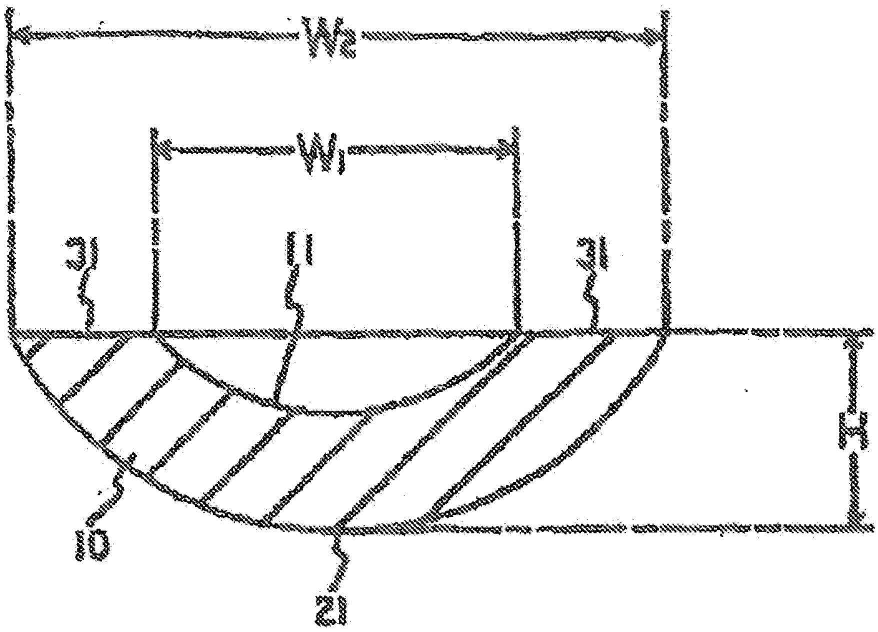


SINGLE FIGURE

EP 2 359 908 A2

## Description

The present invention relates to a composition for topical application, intended, especially in the case of skin displaying dyschromia of the type caused by pigmentation marks, red blotches or shadows under the eyes, for lightening or unifying the complexion.

The composition according to the invention may especially be a skincare product or a makeup product, especially a foundation.

It is commonplace for people with coloured skin, or with pigmentation marks, red blotches or shadows under the eyes, to wish to correct these types of cutaneous dyschromia, and for this purpose use cosmetic or dermatological compositions that allow the complexion to be lightened and unified. To this end, it is known practice to use cosmetic compositions containing bleaching agents. However, it is necessary, to use these agents for a prolonged period and in large amounts in order to observe a bleaching effect on the skin. Furthermore, an effect is not observed immediately on application of the compositions comprising them.

For the purpose of immediate lightening and unifying of the complexion, it is also known practice to use cosmetic compositions containing fluorescent compounds, with a particular focus on optical brighteners, However, these compounds afford an immediate lightening effect only under optimum lighting conditions (natural light of strong intensity).

For the purpose of immediate lightening and unifying of the complexion, it is also known practice to use covering products, which, although hiding skin imperfections, have the major drawback of masking the natural appearance of the skin (mask sensation).

It is also known practice to use products containing interference pigments, which, although being able to hide skin imperfections, have the major drawback of giving the skin an unnatural shiny appearance. Furthermore, these pigments have the particular feature of giving a pearlescent appearance and of substantially colouring the products containing them.

Finally, it is known practice to use products containing a combination of nacre and of matting fillers. However, it is not possible to introduce large amounts of nacres, since this results in pearlescing effects of the formulation, but most of all highly artificial shine on the skin. The dyschromia-correcting effects are thus limited to barely perceptible marks and imperfections.

Thus, one of the objects of the present invention is to provide a non-covering cosmetic composition that has an immediate lightening or unifying effect on the complexion, even on pronounced dyschromia, and which does not give the skin a shiny and/or unnatural appearance.

More specifically, the invention relates to a composition comprising, in a physiologically acceptable medium, concave or annular particles, especially in the form of hollow sphere portions, the said particles comprising a matrix that is coloured in the bulk.

The term "matrix that is coloured in the bulk" means that the matrix has a modified colour by incorporating therein a dye, or several dyes as a mixture.

The term "physiologically acceptable medium" means a non-toxic medium that may be applied to the skin, the lips, the hair, the eyelashes, the eyebrows or the nails. The composition of the invention may especially constitute a cosmetic or dermatological composition.

By virtue of the presence of the concave or annular coloured particles, a lightening, unifying effect is observed after application of the composition to the skin, which smoothes out the grain of the skin. Blackheads are hidden. The complexion is less off-colour. Colour contrasts such as brown spots and shadows under the eyes are attenuated.

According to another aspect, the invention relates to a cosmetic skin-treatment and/or makeup process, especially for lightening and/or unifying the complexion and/or for correcting skin dyschromia, which consists in applying to the skin a composition according to the invention.

According to another aspect, the invention relates to concave or annular particles, especially in the form of hollow sphere portions, comprising a matrix made of a silicone material, the said silicone material being a crosslinked polysiloxane of three-dimensional structure comprising, or consisting of, units of formula (I): $SiO_2$ and of formula (II):

$$R^1SiO_{1-5}$$

in which $R^1$ denotes an organic group having a carbon atom directly bonded to the silicon atom, the said matrix being coloured by the presence of at least one dye.

The particles according to the invention are especially intended to be used in cosmetic compositions according to the invention.

Preferably, the dye is water-soluble or liposoluble.

The invention also relates to the use of a composition according to the invention for lightening and/or unifying the complexion and/or for correcting skin dyschromia.

The compositions according to the invention are preferably non-covering, i.e, they allow the grain of the skin

to show through, while at the same time masking imperfections, and more generally allow skin dyschromia to be corrected.

As a guide, these compositions have a transparency of greater than or equal to 60%.

Such transparency values may be obtained in particular for compositions containing about 3% of coloured particles according to the invention.

Protocol for measuring the transparency of the compositions according to the invention

The composition is spread onto a transparent film (Hp Color laser jet transparency, Hp Invent; CP2936A) using an automatic applicator from Braive Instruments (wet thickness 50 μm)

The spread samples are then placed in a thermostatically regulated and ventilated oven for 24 hours at 37°C.

Once they are dry, these films are placed for evaluation on a contrast card (Prüfkarte type 24/5 - 250 $cm^2$ sold by the company Erichsen). The transparency is then measured by means of a Minolta CR-400 colorimeter using the Y values of the black and white parts obtained in the tristimulus system (X, Y, Z). The transparency value is obtained using the following equation:

$$[1-(Y\ \text{black zone}/Y\ \text{white zone})] \times 100 = \%\ \text{Transparency}$$

If the film is totally opaque, the transparency is equal to 0.

Concave or annular particles of silicone material

The concave or annular particles present in the composition according to the invention may be silicone particles, in particular particles of hollow sphere portions at least partly consisting of a silicone material.

The said particles preferably have a mean diameter of less than or equal to 10 μm, especially ranging from 0.1 μm to 8 μm, preferentially from 0.2 to 7 μm, more preferentially ranging from 0.5 to 6 μm and even more preferably ranging from 0.5 to 4 μm.

The term "mean diameter" means the largest dimension of the particle.

The hollow sphere portions used in the compositions according to the invention may have the form of truncated hollow spheres, with a single orifice communicating with their central cavity, and having a cross section in the form of a horseshoe or an arch.

The silicone material is a crosslinked polysiloxane of three-dimensional structure, it preferably comprises, or even consists of, units of formula (I): $SiO_2$ and of formula (II):

$$R^1SiO_{1.5}$$

in which $R^1$ denotes an organic group having a carbon atom directly bonded to the silicon atom.

The organic group $R^1$ may be a reactive organic group; $R^1$ may more particularly be an epoxy group, a (meth) acryloxy group, an alkenyl group, a mercaptoalkyl, aminoalkyl or haloalkyl group, a glyceroxy group, a ureido group or a cyano group, and preferably an epoxy group, a (meth)acryloxy group, an alkenyl group or a mercaptoalkyl or aminoalkyl group. These groups generally contain from 2 to 6 carbon atoms and especially from 2 to 4 carbon atoms.

The organic group $R^1$ may also be an unreactive organic group; $R^1$ may then more particularly be a $C_1$-$C_4$ alkyl group, especially a methyl, ethyl, propyl or butyl group, or a phenyl group, and preferably a methyl group.

Epoxy groups that may be mentioned include a 2-glycidoxyethyl group, a a 3-glycidoxypropyl group and a 2-(3,4-epoxycyclohexyl)propyl group.

(Meth)acryloxy groups that may be mentioned include a 3-methacryloxypropyl group and a 3-acryloxypropyl group.

Alkenyl groups that may be mentioned include vinyl, allyl and isopropenyl groups.

Mercaptoalkyl groups that may be mentioned include mercaptopropyl and mercaptoethyl groups.

Aminoalkyl groups that may be mentioned include a 3-(2-aminoethyl)aminopropyl group, a 3-aminopropyl group and an N,N-dimethylaminopropyl group.

Haloalkyl groups that may be mentioned include a 3-chloropropyl group and a trifluoropropyl group.

Glyceroxy groups that may be mentioned include a 3-glyceroxypropyl group and a 2-glyceroxyethyl group.

A ureido group that may be mentioned is a 2-ureidoethyl group.

Cyano groups that may be mentioned include cyanopropyl and cyanoethyl groups.

Preferably, in the unit of formula (II), $R^1$ denotes a methyl group.

Advantageously, the silicone material comprises the units (I) and (II) in a unit (I) /unit (II) mole ratio ranging

from 30/70 to 50/50 and preferably ranging from 35/65 to 45/55.

The particles of silicone material may especially be obtained according to a process that involves:

(a) introducing into an aqueous medium, in the presence of at least one hydrolysis catalyst, and optionally of at least one surfactant, a compound (III) of formula $SiX_4$ and a compound (IV) of formula $RSiY_3$, in which X and Y independently denote a $C_1$-$C_4$ alkoxy group, an alkoxyethoxy group containing a $C_1$-$C_4$ alkoxy group, a $C_2$-$C_4$ acyloxy group, an N,N-dilkylamino group containing $C_1$-$C_4$ alkyl groups, a hydroxyl group, a halogen atom or a hydrogen atom, and R denotes an organic group comprising a carbon atom directly bonded to the silicon atom; and

(b) placing the mixture resulting from step (a) in contact with an aqueous solution containing at least one polymerization catalyst and optionally at least one surfactant, at a temperature of between 30 and 85°C, for at least two hours.

Step (a) corresponds to a hydrolysis reaction and step (b) to a condensation reaction.

According to one possible embodiment, the dye(s) is (are) introduced during step a). Alternatively, the dye(s) is (are) introduced at the same time as the polymerization step.

In step (a), the mole ratio of compound (III) to compound (IV) usually ranges from 30/70 to 50/50 and advantageously from 35/65 to 45/55, and is preferentially 40/60. The weight ratio of water to the total amount of compounds (III) and (IV) preferably ranges from 10/90 to 70/30. The order of introduction of compounds (III) and (IV) generally depends on their rate of hydrolysis, The temperature of the hydrolysis reaction generally ranges from 0 to 40°C and usually does not exceed 30°C to avoid premature condensation of the compounds.

For the groups X and Y of compounds (III) and (IV):

as $C_1$-$C_4$ alkoxy groups, mention may be made of methoxy and ethoxy groups;
as alkoxyethoxy groups containing a $C_1$-$C_4$ alkoxy group, mention may be made of methoxyethoxy and butoxyethoxy groups;
as $C_2$-$C_4$ alkyloxy groups, mention may be made of acetoxy and propoxy groups;
as N,N-dialkylamino groups containing $C_1$-$C_4$ alkyl groups, mention may be made of dimethylamino and diethylamino groups;
as halogen atoms, mention may be made of chlorine and bromine atoms.

Compounds of formula (III) that may be mentioned include tetramethoxysilane, tetraethoxysilane, tetrabutoxysilane, trimethoxyethoxysilane, tributoxyethoxysilane, tetraacetoxysilane, tetrapropoxysilane, tetraacetoxysilane, tetra (dimethylamino)silane, tetra-(diethylamino)silane, silane tetraol, chlorosilane triol, dichlorodisilanol, tetrachlorosilane and chlorotrihydrogenosilane Preferably, the compound of formula (III) is chosen from tetramethoxysilane, tetraethoxysilane and tetrabutoxysilane, and mixtures thereof.

The compound of formula (III) leads after the polymerization reaction to the formation of the units of formula (I).

The compound of formula (IV) leads after the polymerization reaction to the formation of the units of formula (II).

The group R in the compound of formula (IV) has the meaning as described for the group $R^1$ for the compound of formula (II).

As examples of compounds of formula (IV) comprising an unreactive organic group R, mention may be made of methyltrimethoxysilane, ethyltriethoxysilane, propyl tributoxysilane, butyltributoxysilane, phenyltrimethoxyethoxysilane, methyltributoxyethoxysilane, methyltriacetoxysilane, methyltripropoxysilane, methyltriacetoxysilane, methyltri (dimethylamino)silane, methyltri(diethylamino)silane, methylsilane triol, methylchlorodisilanol, methyltrichlorosilane and methyltrihydrogenosilane,

As examples of compounds of formula (IV) comprising a reactive organic group R, mention, may be made of:

- silanes containing an epoxy group, for instance 3-glycidoxypropyl trimethoxysilane, 3-glycidoxypropyl triethoxysilane, 2-(3,4-epoxycyclohexyl)ethyl trimethoxysilane, 3-glycidoxypropylmethyl dimethoxysilane, 3-glycidoxypropylmethyl dimethoxysilane, 2-glycidoxyethylmethyl dimethoxysilane, 3-glycicloxypropyl dimethylmethoxysilane and 2-glycidoxyethyl dimethylmethoxysilane;
- silanes containing a (meth)acryloxy group, for instance 3-methacryloxypropyl trimethoxysilane and 3-acryloxypropyl trimethoxysilane;
- silanes containing an alkenyl group, for instance vinyl trimethoxysilane, allyl trimethoxysilane and isopropenyl trimethoxysilane;
- silanes containing a mercapto group, for instance mercaptopropyl trimethoxysilane and mercaptoethyl trimethoxysilane;
- silanes containing an aminoalkyl group, for instance 3-aminopropyl trimethoxysilane, 3-(2-aminoethyl)-aminopropyl trimethoxysilane, N,N-dimethylaminopropyl trimethoxysilane and N,N-dimethylaminoethyl trimethoxysilane;
- silanes containing a haloalkyl group, for instance 3-chloropropyl trimethoxysilane and trifluoropropyl trimethoxysilane;

- silanes containing a glyceroxy group, for instance 3-glyceroxypropyl trimethoxysilane and bis(3-glyceroxypropyl) dimethoxysilane;
- silanes containing a ureido group, for instance 3-ureidopropyl trimethoxysilane, 3-ureidopropyl methyldimethoxysilane and 3-ureidopropyl dimethylmethoxysilane;
- silanes containing a cyano group, for instance cyanoPropyl trimethoxysilane, cyanopropyl methyldimethoxysilane and cyanopropyl dimethylmethoxysilane.

Preferably, the compound of formula (IV) comprising a reactive organic group R is chosen from silanes containing an epoxy group, silanes containing a (meth)acryloxy group, silanes containing an alkenyl group, silanes containing a mercapto group and silanes containing an aminoalkyl group.

Examples of compounds (III) and (IV) that are preferred for the implementation of this invention are, respectively, tetraethoxysilane and methyltrimethoxysilane.

As hydrolysis and polymerization catalysts, it is possible to use, independently, basic catalysts such as sodium hydroxide, potassium hydroxide, sodium carbonate, sodium hydrogen carbonate, aqueous ammonia or amines such as trimethylamine, triethylamine or tetramethylammonium hydroxide, or acidic catalysts such as organic acids, for instance citric acid, acetic acid, methanesulfonic acid, p-toluenesulfonic acid, dodecylbenzenesulfonic acid or dodecylsulfonic acid, or mineral acids such as hydrochloric acid, sulfuric acid or phosphoric acid.

When it is present, the surfactant used is preferably a nonionic or anionic surfactant or a mixture of the two. Sodium dodecylbenzenesulfonate may be used as anionic surfactant. The end of hydrolysis is marked by the disappearance of the water-insoluble products (III) and (IV), and the production of a homogeneous liquid layer.

The condensation step (b) may use the same catalyst as the hydrolysis step or another catalyst chosen from those mentioned above.

After this process, a suspension in water of fine organosilicon-based particles is obtained, which may then optionally be separated from their medium. The process described above can thus include an additional filtration step, for example through a membrane filter, of the product resulting from step (b), optionally followed by a step of centrifugation of the filtrate, which is intended to separate the particles from the liquid medium, and then a step of drying the particles, Needless to say, other separation methods may be used.

The form and dimensions of the hollow sphere portions obtained according to the above process will depend especially on the mode of bringing the products into contact in step (b).

A rather basic pH and cold introduction of the polymerization catalyst into the mixture obtained in step (a) will lead to hollow sphere portions in the form of round-bottomed "bowls", whereas a rather acidic pH and dropwise introduction of the mixture obtained in step (a) into the hot polymerization catalyst will lead to hollow sphere portions having a cross section in the form of a "horseshoe".

According to one preferred embodiment of the invention, bowl-shaped, hollow sphere portions are used. These may be obtained as described in patent application JP-2003 128 788.

Horseshoe-shaped hollow sphere portions are described in patent application JP-A-2000 191 789.

The attached Figure 1 illustrates a concave particle in the form of sphere portions with a bowl-shaped cross section. The width W2 corresponds to the diameter of the particles.

As is seen from this Figure, these concave portions are formed (in cross section perpendicular to a plane of the aperture delimited by the hollow sphere portion) from a small inner arc (11), a large outer arc (21) and segments (31) linking the ends of the respective arcs, the width (W1) between the two ends of the small inner arc (11) ranging from 0.01 to 8 μm and preferably from 0.02 to 6 μm on average, the width (W2) between the two ends of the large outer arc (21) ranging from 0.05 to 10 μm and preferably from 0.06 to 8 μm on average and the height (H) of the large outer arc (21) ranging from 0.015 to 8 μm and preferably from 0.03 to 6 μm on average.

The dimensions mentioned above are obtained by calculating the average dimensions of one hundred particles chosen from an image obtained using a scanning electron microscope.

As concave particles in the form of sphere portions that may be used according to the invention, examples that may be mentioned include:

- bowl-shaped particles consisting of a coloured matrix of crosslinked organosilicone (methylsilanol/silicate crosslinked polymer), of width 2.5 μm, of height 1.2 μm and of thickness 150 nm;
- bowl-shaped particles consisting of a coloured matrix of crosslinked organosilicone (methylsilanol/silicate crosslinked polymer), of width 0.8 μm, of height 0.4 μm and of thickness 130 nm;
- bowl-shaped particles consisting of a crosslinked organosilicone matrix (methylsilanol/silicate crosslinked polymer), of width 7 μm, of height 3.5 μm and of thickness 200 nm.

Advantageously, the concave silicone particles have a mean diameter of less than or equal to 5 μm, especially

ranging from 0.1 μm to 5 μm, preferentially ranging from 0.2 to 5 μm; more preferentially ranging from 0.5 to 4 μm and more preferably ranging from 0.5 to 3 μm.

Besides reducing or even eliminating the tacky feel, these particles allow optimization of the glidance, spreading and comfort properties of the composition according to the invention.

The silicone particles of annular form are preferably chosen from those of the type described and synthesized in patent application US-A-2006/0 089 478. To the synthetic process described in the said document, a step is added via which at least one dye is added to the mixture intended to form the matrix. The particles have a mean outside diameter of from 0.05 to 15 μm and a mean inside diameter of from 0.01 to 10 μm; the difference between the mean outside diameter and the mean inside diameter is from 0.04 to 5 μm.

They have a polysiloxane network comprising siloxane units of formulae (1), (2), (3), (4), (5) and (6)

$SiO_{4/2}$ (1)

$Si(OH)_{3/2}$ (2)

$-R_1SiO_{3/2}$ (3)

$R_2SiO_{3/2}$ (4)

$R_3SiO_{3/2}$ (5)

$R_4SiO_{3/2}$ (6)

in which:

- $R_1$ and $R_3$ denote unreactive hydrocarbon-based groups, and especially $R_1$ and $R_3$ denote alkyl, cycloalkyl, aryl, alkylaryl or aralkyl groups, preferably $C_1$-$C_3$ alkyl groups, especially methyl, ethyl and propyl, and preferentially a methyl group,

and $R_2$ and $R_4$ each denote a hydrocarbon-based group chosen from acryloxy, methacryloxy, vinyl and mercapto groups; the mole ratio of siloxane units of formula (1) /siloxane units of formulae (2), (3) , (4), (5) and (6) being from 20/80 to 50/50; the mole ratio of siloxane units of formulae (2), (3) and (4)/siloxane units of formulae (5) and (6) being from 50/50 to 75/25; the mole ratio of siloxane units of formulae (3) and (5)/siloxane units of formulae (4) and (6) being from 20/80 to 60/40.

Acryloxy groups that may be mentioned include 2-methacryloxyethyl and 3-acryloxypropyl groups.

(Meth)acryloxy groups that may be mentioned include 3-methacryloxypropyl and 3-acryloxypropyl groups.

Mercaptoalkyl groups that may be mentioned include mercaptopropyl and mercaptoethyl groups.

Vinyl groups that may be mentioned include allyl, isopropenyl and 2-methylallyl groups.

The concave or annular silicone particles may be present in the composition according to the invention in a content ranging from 0.1% to 15% by weight, preferably ranging from 0.5% to 10% by weight and preferentially ranging from 0.5% to 7.5% by weight relative to the total weight of the composition.

<u>Non-silicone particles of concave form:</u>

Examples of such particles that may be used include particles based on polymethyl methacrylate, as sold under the trade reference Micropearl M310 by the company Matsumoto.

<u>Coloration of the concave or annular particles;</u>

Any kind of dye may be used for colouring the particles used in the compositions according to the present invention.

The colouring agents constituting the particle colouring system may be liposoluble or water-soluble.

The colouring agent may be a natural colouring agent, which is especially water-soluble or liposoluble.

As illustrations of natural water-soluble colouring agents that may be used for colouring the particles used in the compositions according to the invention, mention may be made of caramel, beetroot juice, carmine, betanin (beetroot), cuprous chlorophylline, methylene blue, anthocyanins (enocyanin, black carrot, hibiscus or elder) and riboflavin.

As illustrations of natural liposoluble colouring agents that may be used, mention may be made particularly of Sudan red, β-carotene, carotenoids, lycopene, palm oil, Sudan brown, quinoline yellow, xanthophylls (capsanthin, capsorubin or lutein), and curcumin.

As other natural colouring agents that are most particularly suitable for use in the invention, mention may be made more particularly of anthocyans from flowers or from fruit or derivatives thereof, flavonoids and tannins extracted from native or fermented plants, juglone, lawsone, extracts of fermented soybean, of algae, of fungi or of microorganisms, flavylium salts that are unsubstituted in position 3, as described in patent EP 1 172 091, extracts of *Gesneria fulgens, Blechum procerum* or *Saxifraga,* and pigments that may be obtained by extraction with an organic or aqueous-organic solvent of a culture medium of micromycetes of the *Monascus* type.
Advantageously however, the colouring of the particles is performed using one or more synthetic colouring agents, for example liposoluble or water-soluble colouring agents.
More preferentially, dyes as featured in the table below are used:

| | | |
|---|---|---|
| FD&C Red No. 40 | FD&C Red. No. 4 | D&C Red No. 17 |
| D&C Red No. 3 | D&C Red No. 34 | D&C Red No. 39 |
| D&C Red No. 6 | D&C Red No. 7 | D&C Red No. 27 |
| D&C Red No. 21 | D&C Red No. 22 | D&C Red No. 31 |
| D&C Red No. 28 | D&C Red No. 30 | D&C Red No. 36 |
| D&C Red No. 33 | D&C Red No. 34 | D&C Orange No. 10 |
| D&C Orange No. 11 | D&C Orange No. 5 | D&C Orange No. 4 |
| FD&C Yellow No. 5 | FD&C Yellow No. 6 | Ext. DC Yellow No. 7 |
| D&C Yellow No. 7 | D&C Yellow No. 8 | D&C Yellow No. 11 |
| D&C Yellow No. 10 | FD&C Blue No. 1 | D&C Blue No. 4 |
| FD&C Green No. 3 | D&C Green No. 6 | D&C Green No. 5 |
| D&C Green No. 8 | D&C Brown No. 1 | Ext. DC Violet No. 2 |
| D&C Violet No. 2 | | |

In one particularly preferred embodiment of the invention, the dye is a red dye. A non-limiting list of dyes that may be used for colouring the particles used in the compositions according to the invention is given below.

| **Chemical name** | **INCI Name** | **CI :** |
|---|---|---|
| Disodium salt of ponceau SX | Red 4 | 14700 |
| Disodium salt of fuchsin acid | Red 33 | 17200 |
| Trisodium salt of allura red | Red 40 | 16035 |
| Disodium salt of phloxine | Red 28 | 45410 |
| Caramel | -- | -- |
| Tetrabromotetrachlorofluorescein | Red 27 | 45410 |
| Eosin | Red 21 | 45380 |

The particles according to the invention have matting and soft-focus properties and furthermore have correcting properties. Besides their capacity, due to the coloration of the particles, to correct skin dyschromia, the coloured particles also have matting and soft-focus properties.
Advantageously, the coloured particles are present in the compositions according to the invention in a content ranging from 0.01% to 15% by weight, preferably ranging from 0.5% to 5% by weight and preferentially ranging from 0.5% to 4% by weight relative to the total weight of the composition.

Wetting agents

According to one embodiment of the invention, the compositions also comprise at least one wetting agent.
The term "wetting agents" means any compound which, when introduced into an aqueous solution at 0.05% by weight, makes it possible to reduce the surface tension of water to a value of less than 35 mN/m and preferably less

than 30 mN/m.

The wetting agents in accordance with the invention are preferably chosen from water-soluble silicones comprising at least one terminal or pendent monovalent polyoxyalkylene group, and which, when introduced at 0.05% by weight into an aqueous solution, are able to reduce the surface tension of water to a value of less than 35 mN/m and preferably less than 30 mN/m.

The wetting agents in accordance with the invention are more preferentially chosen from water-soluble silicones comprising at least one polyoxyalkylene group of general formula (a) below

$$R^2_3SiO\ (R^2_2SiO)_p\ (R^2PESiO)_q\ SiR^2_3 \qquad (a)$$

in which

- the radicals $R^2$, which may be identical or different, denote a monovalent hydrocarbon-based radical chosen from alkyl, aryl and aralkyl radicals containing not more than 10 carbon atoms; some of the radicals $R^2$ may also additionally contain an ethylcyclohexylene monoxide group of formula

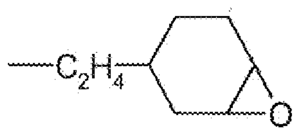

  and are in low proportion in the polysiloxane chain;
- p ranges from 0 to 150, preferably from 0 to 100 and more preferentially from 0 to 30;
- q ranges from 1 to 12, preferably from 1 to 10 and more preferentially from 1 to 8,
- the polyether group PE has the formula (b) below

$$-C_xH_{2x}(OC_2H_4)_y(OC_3H_6)_zOR^3 \qquad (b)$$

  in which:

  x ranges from 1 to 8 and preferably from 2 to 4 and is more preferentially equal to 3;
  y is greater than 0;
  z is greater than or equal to 0; the values of y and z are such that the total molecular weight of the polyoxyalkylene portion of the polyether group PE ranges from 200 to 10 000 and more preferentially from 350 to 4000;
  $R^3$ denotes hydrogen, a $C_1$-$C_8$ alkyl group or a $C_2$-$C_8$ acyl group.

It should be noted that when z is other than 0, the polyoxyethylene and polyoxypropylene units may be randomly distributed along the polyether chain PE or distributed in blocks, or alternatively distributed both in blocks and randomly.

Preferably, the radicals $R^2$ are chosen from methyl, ethyl, butyl, hexyl, phenyl and benzyl groups. More particularly, the radicals $R^2$ are chosen from $C_1$-$C_4$ alkyls and even more particularly denote a methyl radical.

Preferably, the radicals $R^3$ are chosen from $C_1$-$C_4$ alkyls and even more particularly denote a methyl radical.

The number of oxyethylene units in the group PE should be sufficient to produce a cloud point in water of between 25 and 90°C and more preferentially from 40 to 70°C,

The water-soluble silicones of formula (a) may be obtained according to the process described in document US-A-4 847 398.

Among the water-soluble silicones of formula (a) that are preferably used are those of formula (a') below:

$$MeSiO(MeSiO)_p(MePESiO)_qSiMe_3 \qquad (a')$$

in which Me denotes a methyl radical; PE denotes:

$$-(CH_2)_3O(OC_2H_4)_y(OC_3H_6)_zOR^3 \qquad (b')$$

in which y and z have the same values indicated above and $R^3$ denotes hydrogen or a $C_1$-$C_4$ alkyl group, and more particularly a methyl radical.

As another family of water-soluble silicones that may be used according to the invention, mention may be made of the branched silicones of formula (c) below:

$$(MeSiO)_{q-2}[(SiOMe_2)_{p/q}OPE]_q \quad (c)$$

in which p and q have the same values indicated above in formula (a); Me means methyl; PE denotes the group of formula (d) below:

$$-(OC_2H_4)_y(OC_3H_6)_zR^3 \quad (d)$$

in which y and z have the same values indicated above in formula (b) and $R^3$ denotes a $C_1$-$C_4$ alkyl group and more particularly a methyl radical.

Such silicones are sold, for example, by the company OSI under the trade names Silwet L-720®, Silwet L-7002®, Silwet L-7600®, Silwet L-7604®, Silwet L-7605®, Silwet L-7607®, Silwet 1614, Silwet L-7657®,

Silwet Ln 7200®, Silwet L-7230, Sílsoft 305, Silsoft 820 and Silsoft 880, or by the company Goldschmidt under the trade names Tegowet 260, Tegowet 500, Tegowet 505 and Tegowet 510®.

The table below collates the surface tension values at 25°C of aqueous solutions comprising 0.05% (by weight) of various wetting agents.

| Wetting agent | Surface tension at 0.05% in water (mN/m), 25°C |
|---|---|
| Tegowet 500 | 33 |
| Tegowet 510 | 29 |
| Silsoft 880 | 26 |
| Silsoft 305 | 21 |

According to the invention, the wetting agent(s), when it is (they are) present, may be present in concentrations ranging from 0,01% to 10% by weight, preferably from 0.05% to 5% by weight and more particularly from 0.1% to 3% by weight relative to the total weight of the composition.

Dispersants

According to one particular embodiment of the invention, the composition of the invention also contains at least one dispersant. The term "dispersant" means any compound that allows total dispersion in water of the particles used in the composition of the present patent application. The term "total dispersion" means that the particles disperse in water without forming lumps at the surface, and distribute homogeneously, Since the surface of the particles is sparingly lipophilic, it may be advantageous to add a dispersant that will facilitate the dispersion of the particles, especially when the composition is a very fluid emulsion, or even an aqueous lotion.

Preferably, the dispersants are chosen from polyvinyl alcohol and block copolymers of ethylene oxide and propylene oxide.

The block copolymers of ethylene oxide and propylene oxide may be chosen in particular from poloxamers, and especially from Poloxamer 231, such as the product sold by the company BASF under the name Pluronic L81; Poloxamer 282, such as the product sold by the company BASF under the name Pluronic L92; and Poloxamer 124, such as the product sold by the company BASF under the name Pluronic L44.

Matting fillers

According to one embodiment of the invention, the compositions also comprise at least one matting filler.

For the purposes of the invention, the term "matting filler" denotes a spherical or non-spherical, porous or non-porous particle with a refractive index of less than or equal to 2.2, especially less than or equal to 2 and in particular less than or equal to 1.8, preferably ranging from 1.3 to 1.6. The "matting fillers" according to the invention have a volumetric size of less than 15 μm.

In one preferential mode of the invention, the "matting fillers" are spherical.

In one preferential mode of the invention, the "matting fillers" are porous. In this case, the specific surface area of the particles, which may be related to the porosity, is greater than 10 $m^2/g$ and preferably greater than 50 $m^2/g$.

More particularly, these fillers may be chosen, for example, from:

- porous silica microparticles, for instance the silica beads SB150 and SB700 from Miyoshi with a mean size of 5 μm; the series-H Sunspheres from Asahi Glass, for instance Sunspheres H33, H51 and H53 with respective sizes of 3, 5 and 5 μm;

- polytetrafluoroethylene powders, for instance the PTFE Ceridust 9205F from Clariant, with a mean size of 8 μm;
- silicone resin powders, for instance the silicone resin Tospear1 145A from GE Silicone, with a mean size of 4.5 μm;
- uncoloured hollow hemispherical silicone particles such as NLK 500, NLK 506 and NLK 510 from Takemoto Oil and Fat;
- acrylic copolymer powders, especially of polymethyl (meth) acrylate, for instance the PMMA particles Jurymer MBI from Nihon Junyoki, with a mean size of 8 μm, the hollow PMMA spheres sold under the name Covabead LH85 by the company Wackherr, polymethyl methacrylate microspheres sold under the name Microsphere M-100 by the company Matsumoto; or the microspheres sold under the name Micropear1 F 80 ED by the company Matsumoto;
- wax powders, for instance the paraffin wax particles MicroEase 114S from MicroPowders, with a mean size of 7 μm;
- polyethylene powders, especially comprising at least one ethylene/acrylic acid copolymer, and in particular consisting of ethylene/acrylic acid copolymers, for instance the Flobeads EA 209 particles from Sumitomo (mean size of 10 μm);
- crosslinked elastomeric organopolysiloxane powders coated with silicone resin and especially with silsesquioxane resin, as described, for example, in patent US 5 538 793. Such elastomer powders are sold under the names KSP-100, KSP-101, KSP-102, KSP-103, KSP-104 and KSP-105 by the company Shin-Etsu;
- talc/titanium dioxide/alumina/silica composite powders, for instance those sold under the name Coverleaf AR-80 by the company Catalyst & Chemicals;
- polyamide (Nylon®) powders, for instance the Nylon 12 particles of the Orgasol type from Atofina, with a mean size of 10 μm;
- microspheres based on acrylic copolymers, such as those made from ethylene glycol dimethacrylate/lauryl methacrylate copolymer sold by the company Dow Corning under the name Polytrap;
- expanded powders, such as hollow microspheres and especially microspheres formed from a terpolymer of vinylidene chloride, of acrylonitrile and of methacrylate and sold under the name Expancel by the company Kemanord Plast under the references 551 DE 12 (particle size of about 12 μm and mass per unit volume of 40 $kg/m^3$), 551 DE 20 (particle size of about 30 μm and mass per unit volume of 65 $kg/m^3$), 551 DE 50 (particle size of about 40 μm),
- ethylene-acrylate copolymer powders, for instance those sold under the name Flobeads by the company Sumitomo Seika Chemicals;
- powders of natural organic materials such as starch powders, especially of crosslinked or non-crosslinked corn starch, wheat starch or rice starch, such as starch powders crosslinked with octenyl succinate anhydride, sold under the name Dry-Flo by the company National Starch;
- cellulose microbeads and fibres,
- and mixtures thereof.

The composition according to the invention may also contain various additional fillers of mineral or organic origin. They may be of any form, especially platelet-shaped, spherical or oblong, irrespective of their crystallographic form (for example leaflet, cubic, hexagonal, orthorhombic, etc.).

Among the additional fillers that may be used in the composition according to the invention, mention may be made especially of talc, mica, kaolin, poly-α-alanine and polyethylene particles, lauroyllysine, starch, boron nitride, precipitated calcium carbonate, magnesium carbonate, magnesium hydrogen carbonate, barium sulfate, hydroxyapatite, glass or ceramic microcapsules and metal soaps derived from organic carboxylic acids containing from 8 to 22 carbon atoms and especially from 12 to 18 carbon atoms, for example zinc stearate, magnesium stearate, lithium stearate, zinc laurate or magnesium myristate.

The contents introduced depend on the desired effect and range from 0.1% to 20% and preferably from 0.5% to 10%.

## Interference particles

According to one embodiment of the invention, the compositions also comprise at least one interference pigment.

For the purposes of the present invention, the term "interference particles" denotes any particle generally having a multilayer structure such that it allows the creation of a colour effect by interference of light rays that diffract and diffuse according to the nature of the Layers. Thus, these particles may have colours that vary according to the angle of observation and the incidence of the light.

For the purposes of the present invention, a multilayer structure is intended to denote either a structure formed from a substrate covered with a single layer or a structure formed from a substrate covered with at least two or even more consecutive layers.

The multilayer structure may thus comprise one or even at least two layers, each layer, independently or otherwise of the other layer(s), being made of at least one material chosen from the group consisting of the following materials: $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolite, alloys and polymers, and combinations thereof.

Generally, the multilayer structure is of mineral nature.

More particularly, the interference particles under consideration according to the invention may be interference pigments, or alternatively natural or synthetic, monolayer or multilayer nacres, in particular formed from a natural substrate based, inter alia, on mica, which is covered with one or more layers of metal oxide.

The interference particles according to the invention are characterized by a volumetric mean size generally of less than 40 μm, more particularly less than 30 μm, especially less than 20 μm and in particular less than 15 μm, measured with a laser granulometer, for instance the Mastersizer 2000® machine from Malvern and the BI90+® machine from Brookhaven Instrument Corporation.

Nacres of mica/tin oxide/titanium oxide type, for instance those sold under the names Timiron Silk Blue®, Timiron Silk Red®, Timiron Silk Green®, Timiron Silk Gold® and Timiron Super Silk® sold by the company Merck, and mica/iron oxide/titanium oxide nacres, for instance Flamenco Satin Blue®, Flamenco Satin Red® and Flamenco Satin Violet® sold by the company Engelhard, and mixtures thereof, are most particularly suitable for the invention.

It is understood that the choice of these interference particles is made so as to be moreover compatible with the requirements in terms of lightness and saturation required for the compositions according to the invention. In general, these interference particles are present in an amount sufficient to obtain a homogeneous effect in terms of coloration while at the same time preserving the natural flesh tone of the skin and/or the lips.

More specifically, these particles may be present in an amount of less than 15%, more particularly less than 7% and more particularly less than 5% by weight relative to the total weight of the composition.

The compositions according to the invention may be in any galenical form usually used in cosmetics and dermatology, especially in the form of aqueous gels, lotions, emulsions, which may be water-in-oil (W/O), water-in-silicone (W./Si), oil-in-water (O/W), water-in-oil-in-water (W/O/W) or oil-in-water-in-oil (O/W/O) emulsions. These compositions are prepared according to the usual methods.

In addition, the compositions according to the invention are in soft form as opposed to a solid form, i.e. they are not solid. They may be more or less fluid and may have the appearance of a white or coloured cream, an ointment, a milk, a lotion, a serum, a paste or a mousse. They may optionally be in aerosol form.

When the composition according to the invention comprises an oily phase, especially when it is in the form of an emulsion, the oily phase preferably contains at least one oil, especially a physiologically acceptable oil. It may also contain other fatty substances.

As oils that may be used in the composition of the invention, examples that may be mentioned include:

- hydrocarbon-based oils of animal origin, such as perhydrosqualene;
- hydrocarbon-based oils of plant origin, such as liquid triglycerides of fatty acids containing from 4 to 10 carbon atoms, for instance heptanoic or octanoic acid triglycerides, or alternatively, for example, sunflower oil, corn oil, soybean oil, marrow oil, grapeseed oil, sesameseed oil, hazelnut oil, apricot oil, macadamia oil, arara oil, castor oil, avocado oil, caprylic/capric acid triglycerides, for instance those sold by the company Stearineries Dubois or those sold under the names Miglyol 810, 812 and 818 by the company Dynamit Nobel, jojoba oil and shea butter oil;
- synthetic esters and ethers, especially of fatty acids, for instance the oils of formulae $R^1COOR^2$ and $R^1OR^2$ in which $R^1$ represents a fatty acid residue containing from 8 to 29 carbon atoms and $R^2$ represents a branched or unbranched hydrocarbon-based chain containing from 3 to 30 carbon atoms, for instance Purcellin oil, isononyl isononanoate, isopropyl myristate, 2-ethylhexyl palmitate, 2-octyldodecyl stearate, 2-octyldodecyl erucate, isostearyl isostearate or isocetyl stearate; hydroxylated esters, for instance isostearyl lactate, octyl hydroxystearate, octyldodecyl hydroxystearate, diisostearyl malate or triisocetyl citrate; fatty alcohol heptanoates, octanoates or decanoates; polyol esters, for instance propylene glycol dioctanoate, neopentyl glycol diheptanoate and diethylene glycol diisononanoate; and pentaerythriol esters, for instance pentaerythrityl tetraisostearate;
- linear or branched hydrocarbons, of mineral or synthetic origin, such as volatile or non-volatile liquid paraffins, and derivatives thereof, petroleum jelly, polydecenes, and hydrogenated polyisobutene such as Parleam oil;
- fatty alcohols containing from 8 to 26 carbon atoms, for instance cetyl alcohol, stearyl alcohol and a mixture thereof (cetylstearyl alcohol), octyldodecanol, 2-butyloctanol, 2-hexyldecanol, 2-undecylpentadecanol, oleyl alcohol or linoleyl alcohol;
- fluoro oils that are partially hydrocarbon-based and/or silicone-based, for instance those described in document JP-A-2 295 912;
- silicone oils, for instance volatile or non-volatile polymethylsiloxanes (PDMS) with a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end or a silicone chain, these groups containing from 2 to 24 carbon atoms; phenyl silicones, for instance phenyl trimethicones, phenyl dimethicone, phenyltrimethylsiloxydiphenylsiloxanes, diphenyl dimethicones, diphenylmethyldiphenyltrisiloxanes or 2-phenylethyl trimethylsiloxy silicates, and polymethylphenylsiloxanes;

- mixtures thereof.

In the list of oils mentioned above, the term "hydrocarbon-based oil" means any oil mainly comprising carbon and hydrogen atoms, and possibly ester, ether, fluoro, carboxylic acid and/or alcohol groups.
The other fatty substances that may be present in the oily phase are, for example, fatty acids containing from 8 to 30 carbon atoms, for instance stearic acid, lauric acid, palmitic acid and oleic acid; waxes, for instance lanolin wax, beeswax, carnauba wax or candelilla wax, paraffin wax, lignite wax or microcrystalline waxes, ceresin or ozokerite, and synthetic waxes, for instance polyethylene waxes and Fischer-Tropsch waxes; silicone resins such as trifluoromethyl-C1-4-alkyl dimethicone and trifluoropropyl dimethicone.
These fatty substances may be chosen in a varied manner by a person skilled in the art so as to prepare a composition having the desired properties, for example in terms of consistency or texture.
The emulsions generally contain at least one emulsifier chosen from amphoteric, anionic, cationic and nonionic emulsifiers, used alone or as a mixture. The emulsifiers are chosen in an appropriate manner according to the continuous phase of the emulsion to be obtained (W/O or O/W) . When the emulsion is multiple, it generally comprises an emulsifier in the primary emulsion and an emulsifier in the outer phase into which the primary emulsion is introduced.
As emulsifiers that may be used for the preparation of the W/O emulsions, examples that may be mentioned include alkyl esters or ethers of sorbitan, of glycerol or of sugars; silicone surfactants, for instance dimethicone copolyols such as the mixture of cyclomethicone and or dimethicone copolyol, sold under the names DC 5225 C and DC 3225 C by the company Dow Corning, and alkyl dimethicone copolyols such as lauryl methicone copolyol sold under the name "Dow Corning 5200 Formulation Aid" by the company Dow Corning, cetyl dimethicone copolyol sold under the name Abil EM 90® by the company Goldschmidt, and the mixture of Polyglyceryl-4 isostearate/cetyl dimethicone copolyol/hexyl laurate sold under the name Abil WE 09® by the company Goldschmidt. One or more co-emulsifiers may also be added thereto, which may be chosen, advantageously, from the group comprising branched-chain fatty acid esters of polyol, and especially branched-chain fatty acid esters of glycerol and/or of sorbitan, for example polyglyceryl isostearate, such as the product sold under the name Isolan GI 34 by the company Goldschmidt, sorbitan isostearate, such as the product sold under the name Arlcacel 987 by the company ICI, sorbitan glyceryl isostearate, such as the product sold under the name Arlacel 986 by the company ICI, and mixtures thereof.
As emulsifiers that may be used for the preparation of the O/W emulsions, examples that may be mentioned include nonionic emulsifiers such as oxyalkylenated (more particularly polyoxyethylenated) fatty acid esters of polyols, for example polyethylene glycol stearates, for instance PEG-100 stearate, PEG-50 stearate and PEG-40 stearate; and mixtures thereof such as the mixture of glyceryl monostearate and of polyethylene glycol stearate (100 EO) sold under the name Simulsol 165 by the company SEPPIC; oxyalkylenated fatty acid esters of sorbitan comprising, for example, from 20 to 100 EO, for instance those sold under the trade names Tween 20 (INCI name: Polysorbate 20), Tween 60 (INCI name: Polysorbate 60) or Tween 61 (INCI name: Polysorbate 61) by the company Uniqema; oxyalkylenated (oxyethylenated and/or oxypropylenated) fatty alkyl ethers; sugar esters, for instance sucrose stearate and sucrose distearate (INCI name: sucrose distearate), for instance the product sold under the names Crodesta F-10, F-20, F-50, F-70, F-110 and F-140 by the company Croda; and mixtures of these emulsifiers, for instance the mixture of glyceryl stearate and of PEG-100 stearate sold under the name Arlacel 165 by the company Uniqema. Anionic surfactants may also be incorporated, such as amino acid derivatives, for instance the disodium salt of N-stearoyl-L-glutamic acid (INCI name: disodium stearoyl glutamate) sold under the name Amisoft HS-21 by the company Ajinomoto.
Co-emulsifiers may be added to these emulsifiers, for instance fatty alcohols containing from 8 to 26 carbon atoms, for instance cetyl alcohol, stearyl alcohol and the mixture thereof (cetearyl alcohol), octyldodecanol, 2-butyloctanol, 2-hexyldecanol, 2-undecylpentadecanol or oleyl alcohol.
The composition according to the invention may also contain an advantageous amount of amphiphilic polymers as emulsifier or coemulsifier.
The term "amphiphilic polymer" means any polymer comprising both a hydrophilic portion and a hydrophobic portion and having the property of forming a film that separates two liquids of different polarity and thus making it possible to stabilize liquid-liquid dispersions of direct, inverse or multiple type. The amphiphilic polymers that are more particularly suitable reduce the water/oil interface tension to 10 mN/m, irrespective of the oil. These polymers are ionic (anionic or cationic) or amphoteric. They may be water-soluble or water-dispersible. The term water-soluble means that they can be dispersed in water in the form of a molecular solution. The term water-dispersible means that they can be dispersed in water in particulate form.
The amphiphilic polymers that are suitable for use in the invention generally have a number-average molecular weight ranging from 1000 to 20 000 000 g/mol, preferably ranging from 20 000 to 8 000 000 and even more preferentially from 100 000 to 700 000 g/mol. The amounts of amphiphilic polymers used according to the invention will be chosen from 0.01% to 20%, preferably from 0.1% to 10% and even more preferentially from 0.2% to 5% by weight.
Acrylate/C10-C30-alkylacrylate copolymers such as the products sold under the names Pemulen TR1®, Pemulen TR2® and Carbopol 1382® by the company Goodrich, or mixtures thereof, may be used more particularly. The

acrylate/steareth-20 itaconate copolymers and acrylate/ceteth-20 itaconate copolymers sold under the names Structure 2001® and Structure 3001® by the company National Starch may also be used. Among the crosslinked or non-crosslinked amphiphilic AMPS polymers that are most particularly suitable are the products sold under the names Aristoflex LNC® Aristoflex SNC®and Aristoflex HMS® by the company Clariant.

As terpolymers that may be used, mention may be made of the methacrylic acid/methyl acrylate/behenyl dimethyl m-isopropenylbenzylisocyanate terpolymer ethoxylated with 40 OE, i.e. comprising 40 oxyethylene groups, sold by the company Amerchol under the name Viscophobe DB 1000 NP3-NP4. Mention may also be made of crosslinked terpolymers of methacrylic acid, of ethyl acrylate and of polyethylene glycol (10 OE) stearyl ether (Steareth 10), especially those sold by the company Allied Colloids under the name Salcare SC 80.

The anionic polymers that may be used according to the invention are, for example, isophthalic acid or sulfoisophthalic polymers, and in particular the phthalate/sulfoisophthalate/glycol copolymers (for example diethylene glycol/phthalate/isophthalate/1,4-cyclohexanedimethanol) sold under the names Eastman AQ Polymer (AQ35S, AQ38S, AQ55S, AQ48 Ultra) by the company Eastman Chemical,

Emulsions may also be prepared without emulsifiers, stabilized with silicone particles or coated or uncoated metal oxide particles such as $TiO_2$ or the like.

In a known manner, the composition of the invention may also contain adjuvants that are common in cosmetics or dermatology, such as hydrophilic or lipophilic gelling agents, hydrophilic or lipophilic active agents, preserving agents (for example phenoxyethanol and parabens), antimicrobial agents, for instance caprylyl glycol, antioxidants, solvents, fragrances, fillers, bactericides; odour absorbers, dyestuffs and salts. The amounts of these various adjuvants are those conventionally used in the field under consideration, for example from 0.01% to 20% of the total weight of the composition. Depending on their nature, these adjuvants may be introduced into the fatty phase or into the aqueous phase.

Among the active agents that may be used in compositions according to the present invention, mention may be made of:

- keratolytic or pro-desquamating agents, for example α-hydroxy acids, β-hydroxy acids, α-keto acids or β-keto acids, retinoids and esters thereof, retinal, and retinoic acid and derivatives thereof;
- C glycosides and derivatives thereof;
- adenosine and derivatives thereof;
- vitamins, for instance vitamin B3 or PP, B5, E and K1 and derivatives of these vitamins, and especially esters thereof;
- free-radical scavengers;
- sunscreens;
- moisturizers, for instance polyols; urea and derivatives thereof;
- ceramides;
- DHEA and derivatives thereof;
- coenzyme Q10;
- bleaching and depigmenting agents, for instance kojic acid, para-aminophenol derivatives and arbutin and derivatives thereof, and mixtures thereof.

These active agents may be present in an amount ranging, for example, from 0.01% to 15% by weight and preferably from 0.01% to 10% by weight relative to the total weight of the composition.

Needless to say, a person skilled in the art will take care to select the optional additive (s) to be added to the composition according to the invention and the amounts thereof, such that the advantageous properties intrinsically associated with the composition in accordance with the invention are not, or are not substantially, adversely affected by the envisaged addition.

The compositions according to the invention may be in liquid form (for example lotions) or in the form of more or less fluid creams. They may be used for any cosmetic or dermatological application, especially for caring for and making up human skin, both facial and/or bodily skin, more particularly as a foundation (fluid or creamy foundations).

The examples that follow serve to illustrate the invention without, however, being limiting in nature. The amounts are expressed as weight percentages of starting material. The compounds of the compositions according to the invention are, where appropriate, cited as the chemical names or as the INCI (International Cosmetic Ingredient) names.

Example 1: cosmetic composition (oil-in-water emulsion)

| | | |
|---|---|---|
| A: | Glyceryl stearate (and) PEG-100 stearate: | 2.00 g |
| | Dimyristyl tartrate (and) cetearyl alcohol | 1.50 g |

(continued)

| | | |
|---|---|---|
| | (and) C12-15 Pareth-7 (and) PPG-25 Laureth-25: | |
| | Cyclohexasiloxane: | 10.00 g |
| | Stearyl alcohol: | 1.00 g |
| B: | Water: | 80.75 g |
| | Phenoxyethanol: | 1.00 g |
| | Pentasodium ethylenediaminetetramethylene-phosphate: | 0.05 g |
| | Ammonium polyacryldimethyltauramide: | 0.40 g |
| | Xanthan gum: | 0.20 g |
| C: | Hollow hemispherical particles containing a red dye (D&C Red 4): | 3.00 g |

Procedure:

Phase B was heated to about 75°C and the ammonium polyacryldimethyltauramide was incorporated therein. The mixture was stirred until a homogeneous gel was obtained.

Phase A was heated to about 75°C. The emulsion was prepared by incorporating phase A into phase B. At 4.0-45°C, phase C was incorporated and stirring was continued until the emulsion was completely cool.

On individuals with dry skin displaying dyschromia (pigmentation marks, depigmentation, freckles, red blotches or shadows under the eyes), the compositions of Example 1 are claimed to have effects of lightening and unifying the skin, smoothing the grain of the skin, and also hiding blackheads. The complexion will be less off-colour and the colour contrasts such as brown spots and shadows under the eyes will be attenuated.

Example 2: cosmetic composition (water-in-oil emulsion)

| | | |
|---|---|---|
| A: | Oxyethylenated polymethylcetyl dimethyl methylsiloxane: | 1.5 g |
| | Polyglyceryl isostearate: | 0.50 g |
| | Isohexadecane: | 4.00 g |
| | Squalane: | 1.85 g |
| | Dimethicone: | 2.05 g |
| | Apricot kernel oil: | 1.1 g |
| | Cyclopentasiloxane: | 9 g |
| | Propyl paraben: | 0.15 g |
| B: | Water: | 71.5 g |
| | Propylene glycol: | 3.00 g |
| | Magnesium sulfate: | 1.75 g |
| | Methyl paraben: | 0.20 g |
| | Preserving agent: | 0.30 g |
| C: | Nylon 12: | 3.00 g |
| D: | Hollow hemispherical particles containing a red dye (D&C Red 4) : | 5.00 g |

Procedure:

Phase A and phase B were separately homogenized at room temperature with stirring. The emulsion was prepared by incorporating phase B into phase A, and phases C and D were incorporated with stirring.

## Claims

1. Cosmetic composition, especially for treating and/or making up the skin, comprising, in a physiologically acceptable medium, concave or annular particles, especially in the form of hollow sphere portions, the said particles comprising a matrix that is coloured in the bulk, and the matrix being made of a non-silicone material, and being especially based on polymethylmethacrylate (PMMA).

2. Cosmetic composition according to claim 1, **characterized in that** the concave or annular particles are present in a content ranging from 0.01% to 15%, preferably from 0.5% to 5% and more preferably from 0.5% to 4% by weight relative to the total weight of the composition.

3. Cosmetic composition according to any one of claims 1. or 2, **characterized in that** the colouring in the bulk of the matrix results from the incorporation therein of at least one water-soluble or liposoluble dye.

4. Cosmetic composition according to the preceding claim, **characterized in that** the dye(s) is (are) introduced into the matrix to a proportion of from 0.1% to 20% and preferably from 1% to 10% by weight relative to the total weight of the particle.

5. Cosmetic composition according to any one of the preceding claims, **characterized in that** the dye(s) is (are) chosen from FD&C Red No. 40; FD&C Red No. 4; D&C Red No. 17; D&C Red No. 3; D&C Red No. 34; D&C Red No. 39; D&C Red No. 6; D&C Red No. 7; D&C Red No. 27; D&C Red No. 21; D&C Red No. 22; D&C Red No. 31; D&C Red No. 28 ; D&C Red No. 30; D&C Red No. 36; D&C Red No. 33; D&C Red No. 34; D&C Orange No. 10; D&C Orange No. 11; D&C Orange No. 5; D&C Orange No. 4; FD&C Yellow No. 5; FD&C Yellow No. 6; Ext. DC Yellow No. 7; D&C Yellow No. 7; D&C Yellow No. 8; D&C Yellow No. 11; D&C Yellow No. 10; FD&C Blue No. 1; D&C Blue No. 4; FD&C Green No. 3; D&C Green No. 6 ; D&C Green No. 5; D&C Green No. 8; D&C Brown No. 1; Ext. DC Violet No. 2; D&C Violet No. 2.

6. Cosmetic composition according to the preceding claim, **characterized in that** the dye(s) is (are) chosen from FD&C Red No. 4, D&C Red No. 33, FD&C Red No. 40, D&.C Red No. 28, caramel, D&C Red No. 27 and D&C Red No. 21.

7. Cosmetic composition according to any one of the preceding claims, **characterized in that** it also contains at least one compound chosen from dyes, pigments, fillers, especially matting fillers, tensioning agents, fragrances, preserving agents, physical and chemical sunscreens, sequestering agents, moisturizers such as polyols and especially glycerol, and pH regulators (acids or bases).

8. Cosmetic composition according to any one of the preceding claims, **characterized in that** it comprises at least one active agent chosen from keratolytic or pro-desquamating agents, for example α-hydroxy acids, β-hydroxy acids, α-keto acids, β-keto acids, retinoids and esters thereof, retinal, retinoic acid and derivatives thereof, C glycosides and derivatives thereof, adenosine and derivatives thereof, vitamins, for instance vitamins B3 or PP, B5, E and K1, and derivatives of these vitamins and especially esters thereof; free-radical scavengers; sunscreens; moisturizers, for instance polyols; ceramides; DHEA and derivatives thereof; coenzyme Q10; bleaching and depigmenting agents, for instance kojic acid, para-aminophenol derivatives, and arbutin and derivatives thereof, and mixtures thereof.

9. Cosmetic composition according to any one of the preceding claims, **characterized in that** it has a transparency of at least 60%.

10. Cosmetic skin-treatment and/or makeup process, which consists in applying to the skin a composition according to any one of Claims 1 to 9.

11. Process for lightening and/or unifying the complexion and/or for correcting skin dyschromia, which consists in applying to the skin a cosmetic composition according to any one of Claims 1 to 9.

12. Use of a cosmetic composition according to any one of Claims 1 to 9, for lightening and/or unifying the complexion and/or for correcting skin dyschromia.

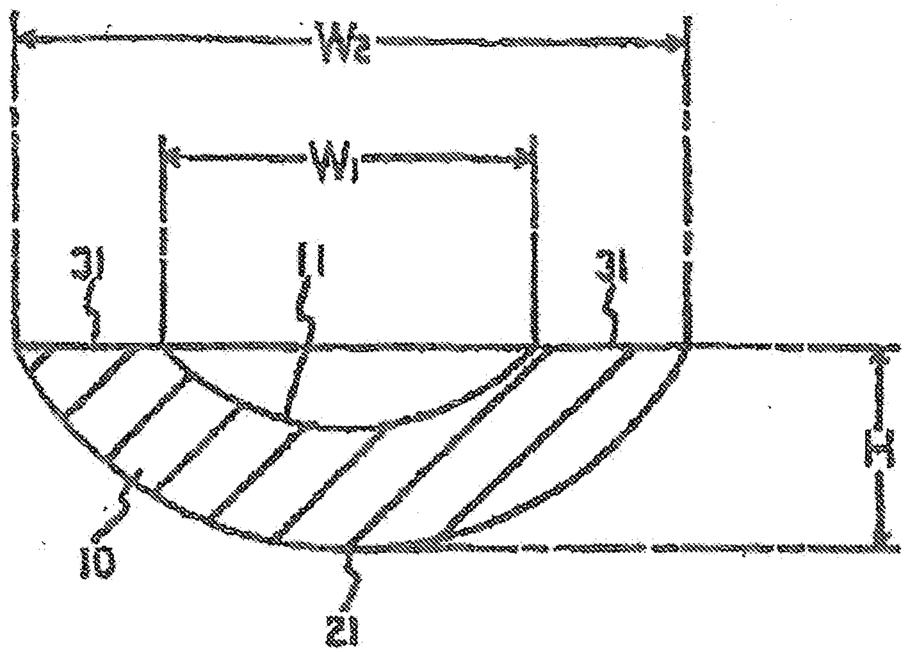


SINGLE FIGURE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003128788 B
- JP 2000191789 A
- US 20060089478 A
- EP 1172091 A
- US 4847398 A
- US 5538793 A
- JP 2295912 A